# EUROPEAN PATENT SPECIFICATION

(11) **EP 2 771 480 B1**
(45) Date of publication and mention of the grant of the patent: **14.12.2016**
(21) Application number: 12791022.2
(22) Date of filing: 29.10.2012
(51) Int. Cl.: C12Q 1/44, C12Q 1/48, G01N 33/53

(54) **METHODS FOR DETECTING ADENOSINE MONOPHOSPHATE IN BIOLOGICAL SAMPLES**
VERFAHREN ZUM NACHWEIS VON ADENOSINMONOPHOSPHAT IN BIOLOGISCHEN PROBEN
PROCÉDÉS DE DÉTECTION D'ADÉNOSINE MONOPHOSPHATE DANS DES ÉCHANTILLONS BIOLOGIQUES

(30) Priority: 28.10.2011 US 201161552837 P
(43) Date of publication of application: 03.09.2014
(73) Proprietor: Promega Corporation, Madison, WI 53711-5399 (US)
(72) Inventor: GOUELI, Said, A., Fitchburg, WI 53711 (US); HSIAO, Kevin, Madison, WI 53717 (US); ZEGZOUTI, Hicham, Madison, WI 53719 (US)
(74) Representative: Potter Clarkson LLP
(86) International application number: PCT/US2012/062397
(87) International publication number: WO 2013/063563

(56) References cited:
- US-A1- 2010 075 350
- Said A Goueli, Kevin Hsiao, Hicham Zegzouti: "Abstract 164:High Throughput Homogenous Bioluminescent Assays For Monitoring The Concentrations of AMP ADP and ATP", Promega , 1 April 2012 (2012-04-01), page 1, XP002695509, America Association for Cancer Research, Annual Meeting, March 31-April 4, 2012, Chicago. Presented at the poster section 6, board 18, on April 1, 2012 . Retrieved from the Internet: URL:http://www.promega.de/~/media/Files/Re sources/Posters/High%20Throughput%20Homoge neous%20Bioluminescent%20Assays%20for%20Mo nitoring%20AMP%20ADP%20and%20ATP%20Poster. pdf [retrieved on 2012-04-15]
- PETER RONNER ET AL: "Luminometric Assays of ATP, Phosphocreatine, and Creatine for Estimation of Free ADP and Free AMP", ANALYTICAL BIOCHEMISTRY, vol. 275, no. 2, 15 November 1999 (1999-11-15), pages 208-216, XP055059787, ISSN: 0003-2697, DOI: 10.1006/abio.1999.4317
- "Technical Manual"; "TM#384" In: "AMP-Glo TM Assay INSTRUCTIONS FOR USE OF PRODUCTS V5011, V5012 AND V5013", 1 October 2012 (2012-10-01), Promega, USA, XP002695713, the whole document
- LUST W D ET AL: "The enzymatic measurement of adenine nucleotides and P-creatine in picomole amounts", ANALYTICAL BIOCHEMISTRY, vol. 110, no. 1, 1 January 1981 (1981-01-01), pages 258-266, XP024818172, ACADEMIC PRESS INC, NEW YORK ISSN: 0003-2697, DOI: 10.1016/0003-2697(81)90144-5 [retrieved on 1981-01-01]
- WEISS B ET AL: "Rapid microassay of adenosine 3',5'-monophosphate phosphodiesterase activity", ANALYTICAL BIOCHEMISTRY, vol. 45, no. 1, 1 January 1972 (1972-01-01), pages 222-235, XP024816323, ACADEMIC PRESS INC, NEW YORK ISSN: 0003-2697, DOI: 10.1016/0003-2697(72)90022-X [retrieved on 1972-01-01]
- RESNICK S M ET AL: "IN VITRO ATP REGENERATION FROM POLYPHOSPHATE AND AMP BY POLYPHOSPHATE: AMP PHOSPHOTRANSFERASE AND ADENYLATE KINASE FROM ACINETOBACTER JOHNSONII 210A", APPLIED AND ENVIRONMENTAL MICROBIOLOGY, vol. 66, no. 5, 1 May 2000 (2000-05-01), pages 2045-2051, XP002937344, AMERICAN SOCIETY FOR MICROBIOLOGY, US ISSN: 0099-2240, DOI: 10.1128/AEM.66.5.2045-2051.2000
- S. TANAKA ET AL: "A sensitive method for detecting AMP by utilizing polyphosphate-dependent ATP regeneration and bioluminescence reactions", BIOCHEMICAL ENGINEERING JOURNAL, vol. 9, no. 3, 1 December 2001 (2001-12-01), pages 193-197, XP055060545, ISSN: 1369-703X, DOI: 10.1016/S1369-703X(01)00144-9

## Description

### Field of the Invention

The present invention relates, in general, to cellular tools and, more particularly, to methods for detecting adenosine monophosphate (AMP) in biological samples.

### Background of the Invention

Adenosine monophosphate (AMP) is an important intracellular mediator of a variety of cellular functions such as the control of the energy charge ratio within a cell, which is an important measurement for balancing anabolic and catabolic processes in the cell. It is also a product of ubiquitination reaction, protein synthesis and translation, nucleic acid ligations, phosphodiesterase activities, etc. Post-translational modifications of proteins by ubiquitin via ubiquitination, small ubiquitin-like modifier (SUMO) via sumoylation, ubiquitin-like protein Nedd8 via neddylation, etc. are key regulatory mechanisms functioning during a diversity of cellular processes such as cell cycle, transcription, nuclear transport, endocytosis, and protein quality control. The ubiquitin-proteasome system (UPS) is responsible for the degradation of many of the pivotal cell signaling molecules. The linkage type of the ubiquitin chain determines whether a modified protein is degraded by the proteasome or serves to attract proteins to initiate signaling cascades or be internalized. The process of ubiquitination is mediated by an enzymatic cascade initiated by ubiquitin activating enzyme (UAE known also as E1) using Adenosine Triphosphate (ATP), ubiquitin or its analogues, ubiquitin conjugating enzyme (Ube2 known also as E2), an ubiquitin ligase (E3) and a substrate to be ubiquitinated. The products of this ubiquitination cascade are AMP, pyrophosphate (PPᵢ), and an ubiquitinated (or polyubiquitinated) substrate. The processes of ubiquitination (or polyubiquitination) and deubiquitination are very attractive areas of research as evidenced by the FDA approval of a proteasome inhibitor VELCADE^{®} for the treatment of patients with multiple myeloma and relapsed mantle cell lymphoma. Thus, targeting other components in the UPS pathway might represent an opportunity to develop novel anticancer therapeutics. Similar interest in the neurodegenerative research of Parkinson diseases identified Nedd4 as the E3 ligase which ubiquitinates alpha synuclein and targets it to the endosomal lysosomal pathway, thus reducing alpha synuclein content and protecting against the pathogenesis of Parkinson diseases and other alpha-synucleinopathies.

Protein translation requires the ligation of substrate amino acids to their cognate tRNAs with high fidelity. This process proceeds in two steps: the activation of amino acids to aminoacyladenylates via consumption of one molecule of ATP and the delivery of activated amino acids to the acceptor end of tRNAs. The fidelity of this process depends on the accuracy of the recognition of amino acids and their cognate tRNAs by aminoacyl tRNA synthase (ARS) and proofreading which prevents ligation of the wrong amino acid to the wrong tRNA. Proofreading occurs before the transfer of aminoacyl-AMP to tRNAs and results in the release of AMP. Thus, the translation of proteins requires a process that consumes ATP, and the release of AMP as the product.Phosphodiesterases (PDEs) are another example of enzymes that use cyclic adenosine 3,5,-monophosphate (cAMP) as a substrate and release AMP as the reaction product. These enzymes are involved in a variety of pathological disorders such as asthma, cardiovascular functions, neurodegenerative disease, etc.

DNA ligases catalyze the ligation of single-stranded breaks to complete DNA replication and repair via ATP breakdown resulting in the formation of a new phosphodiester bond in DNA. Eukaryotic DNA ligases use ATP as the adenylyl group donor, whereas bacterial DNA ligases use either ATP or nicotinamide adenine dinucleotide (NAD+). DNA ligases catalyze the nucleophilic attack of the 3'-hydroxyl on the adenylated 5'-phopshate to form a new phosphodiester bond and release AMP. Bacterial DNA ligases inhibition without affecting eukaryotic ligases or other DNA-binding enzymes have been successfully selected as novel strategy to develop novel antibacterial agents.

Other reactions involving DNA methyltransferases (DMT) and protein methyltransferases (PMT) show promise in the field of epigenetics and are currently under intensive investigation. These enzymes utilize S-adenosyl methionine (SAM) as a substrate, and the resulting product S-adenosylhomocysteine (SAH) can be hydrolyzed to adenosine by SAH hydrolase. The resulting product, adenosine, can be converted to AMP using adenosine kinase. Thus, monitoring AMP formation can be used as readout for DMT and PMT activities.

It was also reported that AMP concentration increases significantly in tumors under hypoxia as compared to wild type, and thus, an increase in the ratio of AMP to ATP causes the allosteric activation of phosphofructokinase-1 (PFK-1). Thus, tumors can maintain normal rate of glycolysis by running at a lower inhibitory ATP concentration and a much higher activating AMP concentration. The higher AMP concentration and lower ATP concentration in tumor cells also cause the activation of AMP-activated protein kinase which coordinates cellular proliferation with carbon source availability. The above examples highlight the significance of monitoring AMP concentrations in biochemical as well as cellular or tissue samples. Validating the targets as well as developing molecule(s) that can alter the biochemical process, which are involved in the development of abnormal cellular physiology, and inducing certain pathologies is dependent on the availability of robust and fast technologies. Thus, there is a need for assays that meet the requirements of a drug discovery program where quality, cost, and speed of the technical work is necessary. Such needs are not met in currently used assays, and thus the AMP detection method of the present invention fulfills theses unmet demands.

### Summary of the Invention

The patent application provides methods to for monitoring AMP in a variety of biochemical and cellular applications. The technology and the kits detect free AMP present in cell extract in the presence of other nucleotides and AMP produced in biochemical reactions such as Phosphodiesterase (PDE), enzymes involved in Ubiquitination (Ligases), amino acyl tRNA Synthetases (ARS), DNA ligases (both bacterial and eukaryotic DNA ligases, etc.

The methods can also detect AMP that is produced indirectly from biochemical reactions by converting their products into AMP and then detection of the newly formed AMP by the current assay format. These include DNA and protein methyltransferases, Protein Isoaspartyl Methyl Transferase (PIMT), etc.

In one embodiment, the invention provides a method of determining the amount of adenosine monophosphate (AMP) in an original solution by converting substantially all AMP in the solution to adenosine diphosphate (ADP) using a first enzyme that is capable of converting AMP to ADP. Suitably at least 90%, or at least 91% or at least 92%, or at least 93%, or at least 94%, or at least 95%, or at least 96%, or at least 97%, or at least 98%, or at least 99%, or at least 99.9% of the AMP in the original solution is converted by the first enzyme. This first enzyme is suitably polyphosphate:AMP phosphotransferase (PAP), but can be any other suitable enzyme. Polyphosphate may optionally be added along with the PAP. The ADP in the solution is then converted into adenosine triphosphate (ATP) using a second enzyme that is capable of converting ADP to ATP. This second enzyme is suitably adenylate kinase (AK), but can be any other suitable enzyme. The conversion of ADP to ATP can be either simultaneous with, or after, the conversion of the AMP to ADP by the first enzyme. The amount of ATP produced is determined by utilizing a luciferase enzyme and a substrate for the luciferase enzyme. Suitably the substrate for the luciferase enzyme is luciferin, but can be any other suitable substrate. The amount of ATP produced can then be used to determine the amount of AMP that was present in the original solution.

In another embodiment the invention provides a method of determining the amount of AMP in an original solution by first converting substantially all ATP in the original solution to cyclic adenosine monophosphate (cAMP) and pyrophosphate using adenylate cyclase (AC), and then converting substantially all AMP in the solution to ADP using a first enzyme that is capable of converting AMP to ADP. The AC can be an active fragment of a full length bacterial adenylate cyclase or an active recombinant fragment of a bacterial adenylate cyclase. Suitably the AC can be a bacterial adenylate cyclase, such as *Bortedella pertussis* or *Bacillus anthracis.* In certain embodiments, suitably at least 90%, or at least 91% or at least 92%, or at least 93%, or at least 94%, or at least 95%, or at least 96%, or at least 97%, or at least 98%, or at least 99%, or at least 99.9% of the ATP in the original solution is converted by AC. The first enzyme used to convert AMP to ADP is suitably PAP, but can be any other suitable enzyme. Polyphosphate may optionally be added along with the PAP. The ADP in the solution is then converted into ATP using a second enzyme that is capable of converting ADP to ATP. Optionally, before the conversion of ADP to ATP, a step of inhibiting the AC in the solution can be done by providing an AC inhibiting compound such as Calmidazolium to the solution. The second enzyme to convert the ADP to ATP is suitably AK, but can be any other suitable enzyme. The conversion of ADP to ATP can be either simultaneous with, or after, the conversion of the AMP to ADP by the first enzyme. The amount of ATP produced is determined by utilizing a luciferase enzyme and a substrate for the luciferase enzyme. Suitably the substrate for the luciferase enzyme is luciferin, but can be any other suitable substrate. The amount of ATP produced can then be used to determine the amount of AMP that was present in the original solution. Optionally, steps of removing substantially all of any pyrophosphate in the solution can be done by including a Pyrophospahatase before the conversion of ADP to ATP and/or before performing the bioluminescent reaction to determine the amount of ATP in the solution. In certain embodiments, suitably at least 90%, or at least 91% or at least 92%, or at least 93%, or at least 94%, or at least 95%, or at least 96%, or at least 97%, or at least 98%, or at least 99%, or at least 99.9% of the pyrophosphate is removed by the Pyrophospahatase.

In another embodiment, the invention provides a method of determining the amount of AMP in an original solution by converting a portion or substantially all cAMP in the original solution to AMP using a phosphodiesterase (PDE) and converting substantially all AMP in the solution to ADP using a first enzyme that is capable of converting AMP to ADP. The conversion of the cAMP to AMP can be before, or simultaneous with, the conversion of AMP to ADP. In certain embodiments, suitably at least 90%, or at least 91% or at least 92%, or at least 93%, or at least 94%, or at least 95%, or at least 96%, or at least 97%, or at least 98%, or at least 99%, or at least 99.9% of the cAMP in the original solution is converted by the PDE. Suitably, the PDE can be PDE 1, PDE 3, PDE 4, PDE 7, PDE 10A, PDE 11, or any other suitable PDE. The first enzyme to convert AMP to ADP is suitably PAP, but can be any other suitable enzyme. Polyphosphate may optionally be added along with the PAP. The ADP in the solution is then converted into ATP using a second enzyme that is capable of converting ADP to ATP. This second enzyme is suitably AK, but can be any other suitable enzyme. The conversion of ADP to ATP can be either simultaneous with, or after, the conversion of the AMP to ADP by the first enzyme. The amount of ATP produced is determined by utilizing a luciferase enzyme and a substrate for the luciferase enzyme. Suitably the substrate for the luciferase enzyme is luciferin, but can be any other suitable substrate. The amount of ATP produced can then be used to determine the amount of AMP that was present in the original solution.

In another embodiment, the invention provides a method of determining the amount of AMP in an original solution by first converting a portion or substantially all ATP present in the original solution to AMP using an ubiquitinating enzyme system (E1, E2, and E3 ligase) and ubiquitin, and then converting substantially all AMP in the solution to ADP using a first enzyme that is capable of converting AMP to ADP. In certain embodiments, suitably at least 90%, or at least 91% or at least 92%, or at least 93%, or at least 94%, or at least 95%, or at least 96%, or at least 97%, or at least 98%, or at least 99%, or at least 99.9% of the ATP in the original solution is converted by the ubiquitinating enzyme. The ubiquitinating enzyme is suitably Ube1 (E1), UbcH5c (E2), CARP2 (E3), or any other suitable ubiquitinating enzyme. This first enzyme for conversion of AMP to ADP is suitably PAP, but can be any other suitable enzyme. Polyphosphate may optionally be added along with the PAP. Simultaneously, or before the conversion of AMP to ADP, substantially all ATP in the original solution can be converted to cAMP and pyrophosphate using adenylate cyclase (AC). The ADP formed by the first enzyme in the solution is then converted into ATP using a second enzyme that is capable of converting ADP to ATP. This second enzyme is suitably AK, but can be any other suitable enzyme. The conversion of ADP to ATP can be either simultaneous with or after the conversion of the AMP to ADP by the first enzyme. The amount of ATP produced is determined by utilizing a luciferase enzyme and a substrate for the luciferase enzyme. Suitably the substrate for the luciferase enzyme is luciferin, but can be any other suitable substrate. The amount of ATP produced can then be used to determine the amount of AMP that was present in the original solution. Optionally, steps of removing substantially all of any pyrophosphate in the solution can be done by including a Pyrophospahatase before the conversion of ADP to ATP and/or before performing the bioluminescent reaction to determine the amount of ATP in the solution.

In another embodiment, the invention provides a method of determining the amount of AMP in an original solution by first converting a portion or substantially all of any ATP present in the original solution to AMP using a DNA ligase, and them converting substantially all AMP in the solution to ADP using a first enzyme that is capable of converting AMP to ADP. In certain embodiments, suitably at least 90%, or at least 91% or at least 92%, or at least 93%, or at least 94%, or at least 95%, or at least 96%, or at least 97%, or at least 98%, or at least 99%, or at least 99.9% of the ATP in the original solution is converted using the DNA ligase. Optionally, the solution may also contain, or have added into it, nicotinamide adenine dinucleotide (NAD+) which is converted by the DNA ligase to AMP. This conversion of NAD+ can be before, or simultaneous with, the conversion of AMP to ADP. The DNA ligase is suitably either a eukaryotic or viral DNA ligase. When NAD+ is present a suitably DNA ligase may be an *E.coli* NAD+ dependant DNA ligase, or other suitable ligase. The first enzyme for conversion of AMP to ADP is suitably PAP, but can be any other suitable enzyme. Polyphosphate may optionally be added along with the PAP. Simultaneously, or before the conversion of AMP to ADP, substantially all ATP in the original solution can be converted to cAMP and pyrophosphate using adenylate cyclase (AC). The ADP formed by the first enzyme in the solution is then converted into ATP using a second enzyme that is capable of converting ADP to ATP. The ADP in the solution is then converted into ATP using a second enzyme that is capable of converting ADP to ATP. This second enzyme is suitably AK, but can be any other suitable enzyme. The conversion of ADP to ATP can be either simultaneous with or after the conversion of the AMP to ADP by the first enzyme. The amount of ATP produced is determined by utilizing a luciferase enzyme and a substrate for the luciferase enzyme. Suitably the substrate for the luciferase enzyme is luciferin, but can be any other suitable substrate. The amount of ATP produced can then be used to determine the amount of AMP that was present in the original solution. Optionally, steps of removing substantially all of any pyrophosphate in the solution can be done by including a Pyrophosphatase before the conversion of ADP to ATP and/or before performing the bioluminescent reaction to determine the amount of ATP in the solution.

In another embodiment, the invention provides a method of determining the amount of AMP in an original solution by converting S-adenosyl methionine (SAM) present in the solution to S-adenosylhomocysteine (SAH) using a methyltransferase; converting the SAH to adenosine using an SAH hydrolase; converting the adenosine to AMP using adenosine kinase; and converting substantially all AMP in the solution to ADP using a first enzyme that is capable of converting AMP to ADP. The conversion of SAM to SAH, SAH to adenosine, and adenosine to AMP can be done before, or simultaneous with, the conversion of AMP to ADP. The methyltransferase suitable can be histone-lysine N-methyltransferase, (DNA (cytosine-5)-methyltransferase, Protein Isoaspartate Methyl Transferase and protein arginine methyltransferase, or any other suitable methyltransferase. The first enzyme for conversion of AMP to ADP is suitably PAP, but can be any other suitable enzyme. Polyphosphate may optionally be added along with the PAP. The ADP in the solution is then converted into ATP using a second enzyme that is capable of converting ADP to ATP. This second enzyme is suitably AK, but can be any other suitable enzyme. The conversion of ADP to ATP can be either simultaneous with or after the conversion of the AMP to ADP by the first enzyme. The amount of ATP produced is determined by utilizing a luciferase enzyme and a substrate for the luciferase enzyme. Suitably the substrate for the luciferase enzyme is luciferin, but can be any other suitable substrate. The amount of ATP produced can then be used to determine the amount of AMP that was present in the original solution.

In another embodiment, the invention provides a method of determining the amount of AMP in an original solution by converting a portion of or substantially all of any ATP in the solution to AMP using an aminoacyl tRNA synthetase and then converting substantially all AMP in the solution to ADP using a first enzyme that is capable of converting AMP to ADP. In certain embodiments, suitably at least 90%, or at least 91% or at least 92%, or at least 93%, or at least 94%, or at least 95%, or at least 96%, or at least 97%, or at least 98%, or at least 99%, or at least 99.9% of the ATP in the original solution is converted using an aminoacyl tRNA synthetase. The first enzyme for conversion of AMP to ADP is suitably PAP, but can be any other suitable enzyme. Polyphosphate may optionally be added along with the PAP. The ADP in the solution is then converted into ATP using a second enzyme that is capable of converting ADP to ATP. This second enzyme is suitably AK, but can be any other suitable enzyme. The conversion of ADP to ATP can be either simultaneous with or after the conversion of the AMP to ADP by the first enzyme. The amount of ATP produced is determined by utilizing a luciferase enzyme and a substrate for the luciferase enzyme. Suitably the substrate for the luciferase enzyme is luciferin, but can be any other suitable substrate. The amount of ATP produced can then be used to determine the amount of AMP that was present in the original solution. Optionally, steps of removing substantially all of any pyrophosphate in the solution can be done by including pyrophosphatase before the conversion of ADP to ATP and/or before performing the bioluminescent reaction to determine the amount of ATP in the solution.

The Biochemical Engineering Journal, vol. 9, no. 3, 1 December 2001, pages 193-197 (S. Tanaka et al: "A sensitive method for detecting AMP by utilizing polyphosphate-dependent ATP regeneration and bioluminescence reactions) describes a sensitive method for detecting AMP by using polyphosphate:AMP phosphotransferase (PPT) and adenylate kinase (ADK) from *Acinetobacterjohnsonii* in conjugation with firefly luciferase. This method provided detection of AMP over the concentration range of 0.3-300 pico mol per assay. Samples taken from various sources susceptible to bacterial contamination were subjected to AMP assay using the method and the results showed that it was able to detect food residues with high sensitivity.

### Brief Description of the Drawings

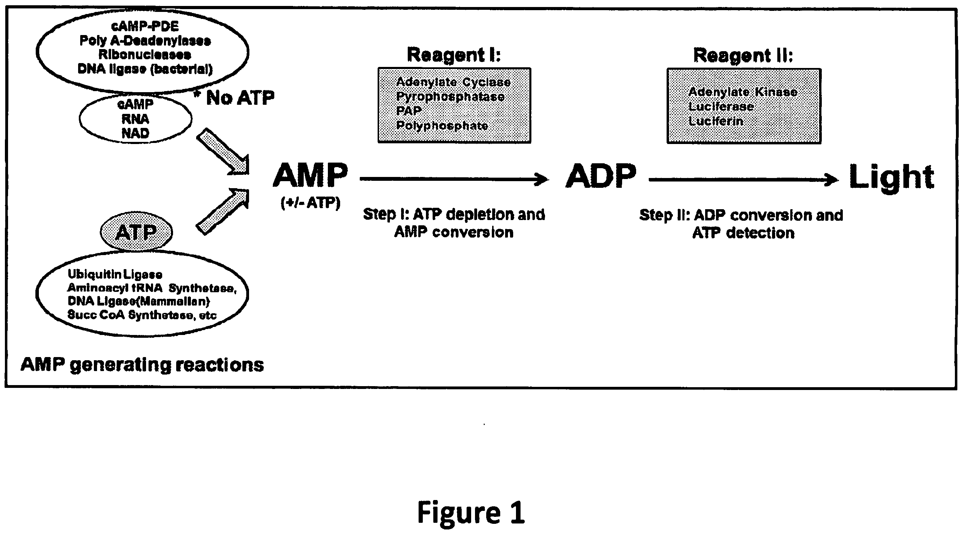

Figure 1 shows a scheme of the AMP detection assay described in the present invention.
Figure 2 illustrates the linearity of AMP detection in the presence or absence of ATP using the method and compositions of the present invention as described in Example 1.
Figure 3 shows a titration of E3 Ligase CARP2 using 100 µM ATP and 40 µM ubiquitin in the presence of 150 nM E1 and 600 nM E2. E3 ligase activity determined using the methods and compositions of the present invention as described in Example 2.
Figure 4 shows a titration of various cAMP Phopshodiesterases. The detection of cAMP PDE activities was carried out using the methods and compositions of the present invention in one or two step assay as described in Example 3.
Figure 5 shows a titration of various inhibitors against PDE 3B using the methods and compositions of the present invention as described in Example 3.
Figure 6 shows a T4 DNA ligase titration detected using the methods and compositions of the present invention as described in Example 4.
Figure 7 shows the specificity of *E. coli* DNA ligase towards various nicotinamide substrates demonstrated using the methods and compositions of the present invention as described in Example 4.

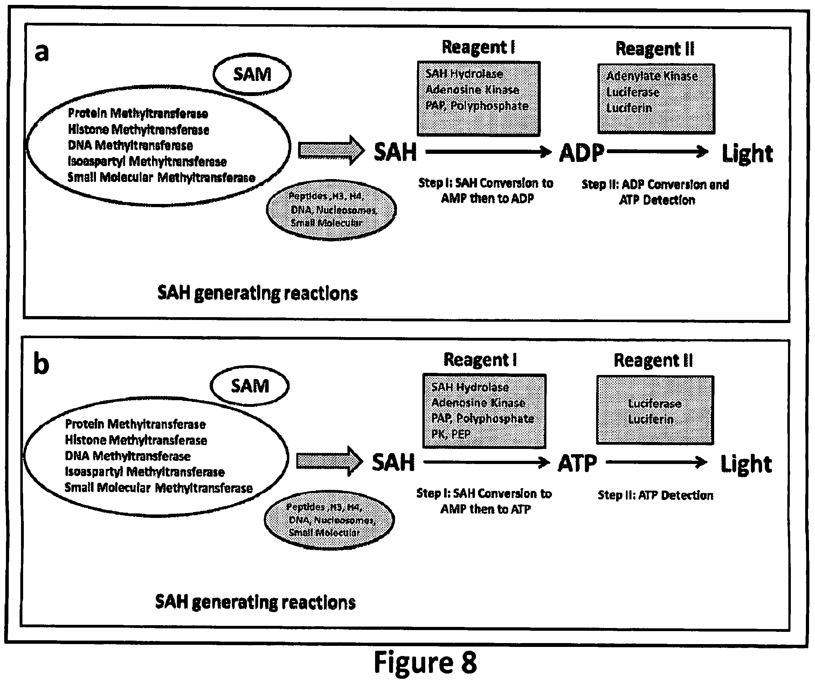

Figure 8 shows a scheme of the AMP detection assay applied to SAH detection for different Methyltransferase activity determinations described in the present invention.
Figure 9 shows a determination of various methyltransferase enzyme activities using the methods and compositions of the present invention as described in Example 6. The invention was used to detect the activity of A) Lysine Methyltransferase (EHMT2-G9a), B) Arginine Methyltransferase (PRMT5), and C) DNA Methyltransferase (DNMT1).
Figure 10 shows a titration of the lysine methyltransferase, EHMT2-G9a, using different substrates and the methods and compositions of the present invention as described in Example 6.

### Detailed Description of the Invention

The present invention provides methods to detect and/or determine the amount and/or presence of adenosine monophosphate (AMP) in biological samples. The present invention also provides compositions and methods to detect or determine the activity of various enzymes, e.g., phosphodiesterases (PDEs), ubiquitin and ubiquitin-like activating enzymes (E1), ubiquitin and ubiquitin-like conjugating enzymes (E2), ubiquitin and ubiquitin-like ligases (E3), DNA ligases, aminoacyl tRNA synthetases (ARS), DNA methyltransferases (DMT) and protein methyltransferases (PMTs), protein isoaspartate methyltransferases, etc. and by detecting and/or determining the amount of AMP in a biological sample. The present invention may also be used to detect AMP in a mixture containing other nucleotides such as adenosine diphosphate (ADP), adenosine triphosphate ATP, guanosine triphosphate (GTP), etc. The method of the present invention can be used to detect or determine the amount of AMP in pure biochemical enzymatic reactions or in a sample of cellular or tissue extracts.

The present invention also provides methods used to detect AMP in any sample that may contain AMP such as extracts of cells or tissues, fluid samples such as urine or blood, or biochemical enzymatic reactions involving purified enzymes such as PDEs, ARS, ubiquitin or DNA ligases, etc. In other embodiments, the methods of the present invention may use an enzyme(s) that converts AMP to ADP and a substrate(s) for this enzyme some embodiments, may also use an enzyme(s) that converts ADP to ATP, and an enzyme that converts ATP into a luminescent signal, i.e., luminescence, via a luciferin-luciferase reaction. The present invention may also use buffer components and substrates such as polyphosphates, magnesium and calmodulin which may be required for the activity of the enzymes present some embodiments, may also use pyrophosphatase which breaks down pyrophosphate generated from enzymes such as adenylate cyclase (AC). In some embodiments, the components for the conversion of ADP to ATP and ATP detection, e.g., luciferin-luciferase reaction can be added separately or simultaneously. In some embodiments, the components for the removal or depletion of ATP and the conversion of AMP to ADP can be added separately or simultaneously.

In some embodiments, wherein methyltransferases such as DNA, protein, and small molecules methyltransferase activity is to be detected or determined, wherein the universal substrate is S-adenosylmethionine (SAM) and the universal product is S-adenosylhomocysteine (SAH), the product SAH is converted to AMP by enzymes such as S-adenosylhomocysteine hydrolase (SAHH) and adenosine kinase (AdK), and the substrate is GTP or dGTP. In some embodiments, for detecting or determining methyltransferase activity additionally use SAHH, AdK and GTP or dGTP. In some embodiments, may also use a standard for methyltransferases such as the substrate S-adenosylmethionine and/or SAH, a standard for monitoring product formation. In some embodiments, the components for the conversion of AMP to ADP, ADP to ATP and the ATP detection, e.g., luciferin-luciferase reaction can be added separately or simultaneously.

In some embodiments, the method of the present invention provides a two-step method, wherein in a first step, ATP is removed using adenylate cyclase (AC) (so minimal or no ATP remains) while simultaneously converting AMP present in the sample to ADP using polyphosphate:AMP phosphotransferase (PAP) and polyphosphates. In a second step, the ADP produced is converted to ATP using adenylate kinase (AK) while simultaneously detecting the ATP formed with an ATP-detection reagent containing luciferin and a luciferase to generate a luminescent signal, i.e., luminescence. The amount of luminescence generated is proportional to the amount of AMP produced in the first reaction. In some embodiments, the AMP detected correlates to the enzymatic activity of enzymes such as PDEs, ARS, DNA ligases, DNA or protein methyltransferases, or protein isoaspartate methyltransferases, or ubiquitin ligases in a sample.

In other embodiments, the method of the present invention can also be used to monitor AMP present in a sample containing ATP and other nucleotide triphosphates. It is noteworthy that in an AC reaction, which uses ATP as substrate, the cAMP generated has insignificant or no effect on the later reactions using AK and luciferase. Since GTP and CTP are the major nucleotide triphosphates in a cell or tissue extracts and biological samples, and both are poor substrates for firefly luciferases, minimal interference to luminescence will occur when these are present in a sample. Similar results can be also obtained when using other enzymes that convert ADP to ATP such as pyruvate kinase with a phosphoenolpyruvate substrate, creatine kinase with a phosphocreatine substrate, and polyphosphate kinase PPK. In some embodiments, an enzyme such as phosphopyruvate dikinase (PPDK) can be used to convert AMP directly to ATP in one step. This reaction, though, has been shown not to be tolerated by the luciferin-luciferase reaction reagents containing luciferase, luciferin, phosphates, detergents, antifoaming agents, etc.

The strength of the methods of the present invention lies in its simplicity, high sensitivity, robustness, no requirement for antibodies, and ease of use. Another advantage of the present invention is that it minimizes the concern over interference from fluorescent compounds and/or other chemicals since the luciferin-luciferase reaction reagent formulation contains a high concentration of luciferin thus minimizing the effect of luciferin chemical analogues. Furthermore, the present invention makes use of polyphosphate, and not ATP, as a phosphate donor for both PAP and AK. This circumvents the problems that occur when using ATP as phosphate donor since ATP will interfere with the luciferin-luciferase reaction as ATP is a substrate for luciferase. Thus, the only source of luminescence is generated from AMP in the sample which is converted to ATP via PAP/AK combination. In some embodiments, the components necessary for the conversion of ADP to ATP and the luciferin-luciferase reaction can be added separately instead of being combined.

In some embodiments, the luciferase enzyme used in the luciferin-luciferase reaction is a beetle luciferase such as firefly luciferase, e.g., *Photinus pyralis, Photuris pennsylvanica,* etc., a recombinant form of a beetle luciferase, a mutant form of a beetle luciferase, e.g., one that is thermostable and/or chemostable, or any luciferase that uses ATP, luciferin, molecular oxygen, magnesium, or any other divalent cation, to generate luminescence can be used in the method of the present invention.

In some embodiments, the adenylate cyclase (AC) can be a recombinant active fragment of the pertussis toxin from *Bortedella pertussis.* In other embodiments, other sources of AC such as those from *V. cholerae,* e.g., cya, *B. anthracis,* mammalian adenylate cyclases and other sources can be used in the method of the present invention. In other embodiments, activators of AC such as calmodulin and divalent cations may also be included in the compositions and methods of the present invention.

### Examples

To create a robust assay for determining AMP concentrations and/or monitoring AMP formation as the universal product of many biochemical reactions, the AMP generated is converted to ADP using PAP while simultaneously depleting ATP if it is the substrate utilized to generate AMP. The depletion of ATP is carried out using a combination of adenylate cyclase and pyrophosphatase. The ADP produced from addition of PAP is converted to ATP using adenylate kinase (AK) while simultaneously converting the ATP generated into a luminescent signal, i.e., luminescence using an ATP detection reagent, e.g., a luciferin-luciferase reaction. The bioluminescent reaction has sufficient sensitivity and steady signal for large batch of plates.

### Materials

Myokinase (AK) from rabbit skeletal muscle (Sigma Catalog number M3003)
Pyruvate kinase from rabbit skeletal muscle (Sigma Catalog number P9136)
Phosphoenolpyruvate (Sigma Cat#7127)
Creatine phosphokinase from bovine heart (Sigma Catalog number C7886)
Creatine phosphokinase from rabbit muscle (Sigma Catalog number C3755
   Adenylate Cyclase (AC) was a recombinant fragment of B. pertussis toxin that contains the active AC portion and demonstrated activity using ATP as substrate. It was obtained from Promega Corp.
AMP 10 mM (Sigma cat# A1752)
Sodium hexametaphosphate or called polyphosphate hereafter (Sigma Cat# P8510)
AMP Glo Reagent I (ATP depletion/AMP to ADP Conversion Reagent): 80mM Tris pH 7.5, 10U/ml calmodulin, 2U/ml pyrophosphatase, 0.1mg/ml Prionex, 60 U/ml HQ-tagged AC, 10mM MgCl2, 8ug/ml HQ tagged-PAP and 40uM polyphosphate
   AMP Glo Reagent II (ADP to ATP Conversion Reagent): 2KU/ml of AK, 4mM polyphosphate, 3.2mM Ammonium sulfate pH 6.0, and 1mM EDTA.
AMP Detection Reagent: contains 10 µl of AMP Glo reagent II in 1 ml of Kinase-Glo® reagent
Kinase Glo Reagent (Promega Corp): Mix Kinase-Glo substrate and Kinase-Glo® Buffer,
   DNA or protein methyltransferases substrate (S-adenosylmethionine) (Promega)
S-adenosylhomocysteine hydrolase (Promega)
Adenosine kinase to convert the enzyme reaction product S-adenosylhomocysteine (SAH) to adenosine and to convert adenosine to AMP. (Promega)

### Methods

### Cloning and Expression of Polyphosphate-AMP Phosphotransferase (PAP):

Genomic DNA of *Acinetobacter johnsonii* 210A was isolated from a bacterial culture, purified, and solubilized in distilled sterilized water. PCR was performed in a reaction containing the primers 5'-AAGTTTAAACTTAACGCCCGCTTTGGTTTA-3' (SEQ. ID 1) and 5'-AAGCGATCGCACGGAACTCGCTATCACAAA-3' (SEQ. ID. 2), a nucleotide mix and Taq DNA polymerase using the isolated genomic DNA as a template. The PCR fragments were purified and cloned into pGEM-T^{®} vector (Promega Corp.), and the recombinant plasmid transformed into JM109 cells and grown on LB plates containing appropriate antibiotic. Well-grown bacterial colonies were selected to grow in culture for plasmid isolation and purification. Plasmid DNA was verified via restriction enzyme digestion. DNA was sequenced using the University of Iowa facility to verify the correct PAP sequence was cloned. PAP DNA was then subcloned into the pFN6K (HQ) Flexi® Expression Vector (Promega Corp.) and transformed into JM109 growing in LB medium containing kanamycin. Plasmids were isolated and digested using restriction enzymes to verify the correct size of the DNA. BL21 (DE3) cells were transformed using the expression vector and grown in LB culture containing kanamycin to an OD₆₀₀ 0.4. The bacterial culture was then induced with IPTG (0.1 mM) and cultured for an additional 2 hrs. before lysis using Fast Break Lysis Buffer (Promega Corp.), and the size (55kDa) verified. Large scale bacterial culture was made, and the bacteria induced and lysed as described above for the small scale culture. PAP enzyme was purified from the cell lysate using HisLink resin (Promega Corp) and eluted using 500 mM imidazole after washing with 350 mM Imidazole to remove extraneous proteins. The size of the purified enzyme was verified via SDS-PAGE. Activity was confirmed using AMP conversion to ADP, and HPLC to verify the generation of ADP and consumption of AMP substrate.

### Activity Assay for PAP:

A standard reaction mixture containing Tris-HCl pH 8.0, 40 mM (NH₄)₂(SO₄), 4 mM MgCl₂, 10 µM AMP and 30 µM polyphosphate, whose average chain length is approximately 750 phosphate residues, was made. The reaction was initiated by the addition of PAP and performed at 37°C. The ADP formed was quantified using HPLC and known AMP, ADP, and ATP standards. Only the product ADP and the remaining AMP were identified on the chromatogram. One unit of PAP is defined as the amount of enzyme producing one micromole of ADP per minute.

### Adenylate cyclase assay

Activity of various adenylyl cyclases (ACs) was measured at 30°C for 30 minutes in the presence of 60 mM Tris pH 7.5, 10 mM MgCl₂, 125 nM calmodulin and variable ATP concentrations. Except for those experiments related to AC titration, 100 ng of the AC was used. When used, calmodulin antagonists were prepared according to the manufacturer's recommendations and were added directly to the AC reaction when direct inhibition was assessed or to the reaction converting ADP to ATP to assess AC inhibition during ATP formation

### AMP Detection Assay

Testing samples containing AMP for AMP concentrations will be dependent on whether the sample contains ATP. If the sample contains ATP, depending on the volume of the reactions and the concentration of ATP, the sample should be treated with sufficient volume and concentration of reagent that contains adenylate cyclase, pyrophosphatase, magnesium, calmodulin, Tris or HEPES buffer pH 7.5, sufficient amount of PAP enzyme (25 ng/25 µL reaction), and polyphosphate (40 µM), e.g., AMP to ADP conversion reagent (AMP Glo Reagent I). The reaction is carried out at room temperature, or any other temperature of choice such as 25, 30, 37, 40°C, etc., for sufficient time to ensure the completion of ATP conversion to cAMP and simultaneous conversion of AMP to ADP. Suitably at least 90%, or at least 91% or at least 92%, or at least 93%, or at least 94%, or at least 95%, or at least 96%, or at least 97%, or at least 98%, or at least 99%, or at least 99.9% of the ATP in the original solution is converted by the AC. Furthermore, suitably at least 90%, or at least 91% or at least 92%, or at least 93%, or at least 94%, or at least 95%, or at least 96%, or at least 97%, or at least 98%, or at least 99%, or at least 99.9% of the AMP in the original solution is converted by the PAP. For the conversion of ADP to ATP while simultaneously converting the ATP into luminescence, an addition of a AMP detection reagent containing myokinase (AK, 1 U/50 µl detection reagent), polyphosphate (40 µM), magnesium chloride (10 mM) or other divalent cations, luciferin, and luciferase and other components can be used. The amount of luminescence generated is proportional to the amount of AMP present in the sample. Alternatively, the conversion of ADP to ATP can be performed separately using a reagent containing myokinase (AK, 1 U/50 µl detection reagent), polyphosphate (40 µM), magnesium chloride (10 mM) or other divalent cations, e.g., ADP to ATP conversion reagent (AMP Glo Reagent II). The ATP can then be measured by the addition of an ATP detection reagent, e.g., a luciferin-luciferase assay reagent, e.g., the Kinase-Glo assay reagent (Promega Corp.). If ATP is not present in the first reaction such as in PDE assays, the removal of ATP is not relevant, and thus conversion of AMP to ADP is directly performed using a reagent that contains PAP, polyphosphate, magnesium, e.g., MgCl₂, and a buffer such as Tris or HEPES pH 7.5. Other pH values such as 7.0, 8.0, and 9.0 or any pH in between can be used in the compositions of the present invention. The ADP formed in the last step is converted to ATP as described above, and the ATP to luminescence using an ATP detection reagent, e.g., a luciferin-luciferase reagent, e.g., Kinase-Glo (Promega Corp.) as described above.

### Example 1: AMP detection assay

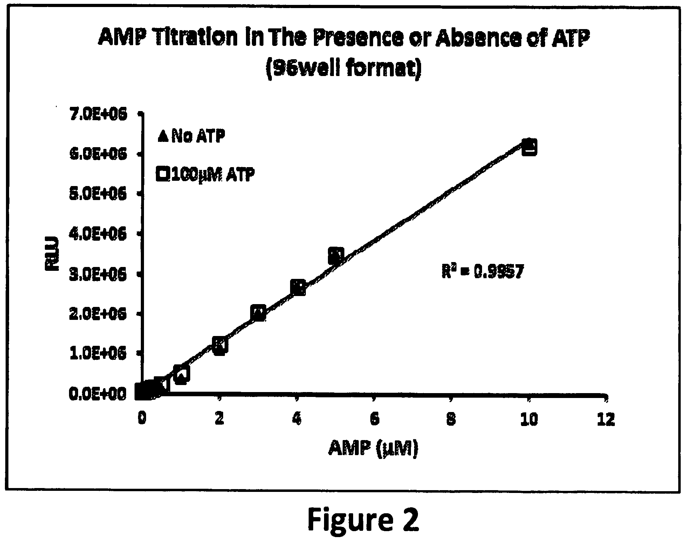

The AMP detection assay is performed generally in two steps as follows. In this example, the AMP titration was performed in 25µl containing Reaction buffer A (40mM Tris-HCl, pH 7.5, 20mM MgCl₂, and 0.1mg/ml BSA) in the presence or absence of 100µM ATP. 25µl of AMP-Glo Reagent I was added to the AMP samples and the mixture was incubated for 60 min at Room Temperature (23°C). To detect AMP, 50µl of AMP Detection Reagent (10µl of AMP-Glo Reagent II in 1ml of Kinase-Glo^{®} One Solution) was added and the mixture was incubated for 60min at Room Temperature (23°C) before luminescence was read in a luminometer. The AMP titration described in Figure 2 was performed in 96well plates (Coming Costar^{®}, cat#3912).

### Example 2: Ubigitinating Enzyme System

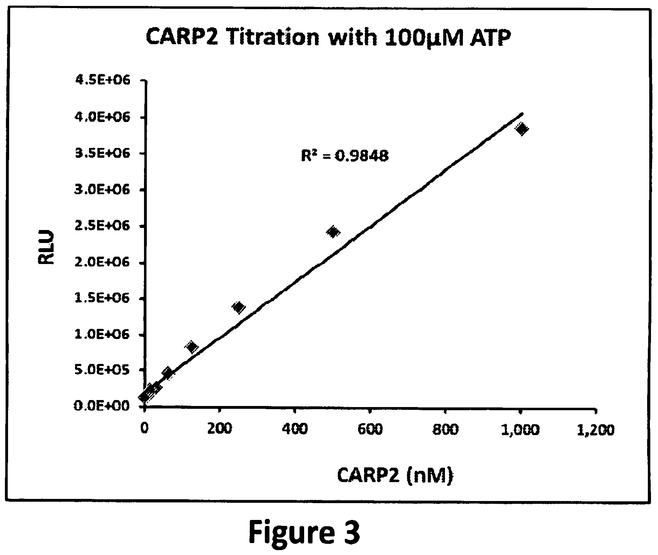

Reaction with an ubiquitinating enzyme or an enzyme involved in an ubiquitinating enzyme system does include the use of ATP as substrate. The method of the present invention was used to assay representative ubiquitinating enzymes including, but not limited to, Ubel (E1), UbcH5c (E2), CARP2 (E3), and ubiquitin was used as a substrate. The enzymes were purchased as a complete kit (E3LITE customizable Ubiquitin Ligase Kit). Each enzyme was titrated separately and together, and the reaction performed in the E3 Ubiquitin Reaction Buffer (Reaction Buffer A supplemented with 0.2mM DTT). AMP was converted using AMP Glo Reagent I followed by the conversion of ADP to ATP and detection of ATP using the AMP Detection Solution. Briefly, 5ul of serially diluted E3-ligase (CARP2; 20x) in E3 Ubiquitin Reaction Buffer was added to wells of a 384-well plate. The CARP2 reaction was started by adding 5µl of a mixture containing E3 Ubiquitin Reaction Buffer, 100µM ATP, 0.15µM ubiquitin E1 activating enzyme, 0.6µM ubiquitin E2 conjugating enzyme, and 40µM recombinant Human ubiquitin. After incubation of 120 minutes at 23°C, 10µl of the AMP to ADP conversion reagent was added, mixed for 2 minutes on an orbital shaker, and then incubated at room temperature for 60 minutes. To the samples, 20µl of AMP Detection Reagent was added, and the samples were incubated at room temperature for 60 minutes. Luminescence was then detected on a lumimometer (Figure 3). It was found that AMP generated in the reaction was proportional to the ubiquitination, conjugation and/or ligase enzyme activity. No activity was found when ubiquitin was not present. Thus, the method of the present invention can be used to screen for compounds that alter the activity of the all enzymes involved in ubiquitination or any of the enzymes involved in an ubiquitating enzyme system.

### Example 3: cAMP Phosphodiesterases (PDEs):

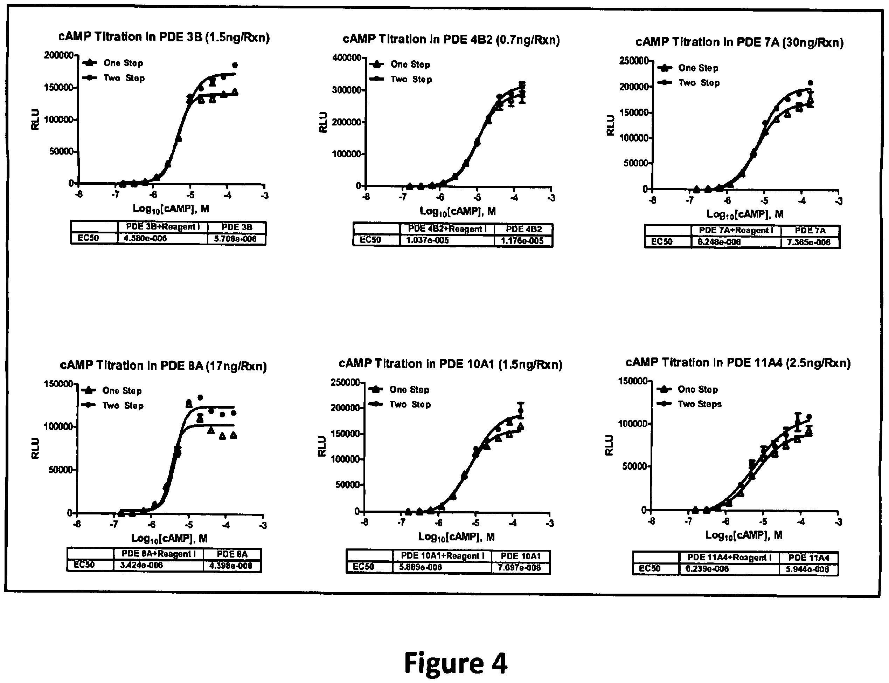

PDE enzymes do not use ATP as substrate, but instead use cAMP. In the method of the present invention, reactions with PDE enzymes, including but not limited to PDE 4, PDE 1, 2, 3, PDE 10A and PDE 11, were carried out at concentrations up to 100 µM cAMP, and the amount of luminescence generated was proportional to substrate concentration until saturation was reached and was proportional to the amount of enzyme within the linear range of the reaction. AMP in the samples was converted to ADP using a AMP-Glo Reagent I supplied with Isobutylmethylxanthine (IBMX). IBMX is known to inhibit most of PDEs and thus it terminates the PDE reaction. Its inclusion in the AMP Glo reagent should result in termination of PDE reaction and conversion of AMP produced into ADP. The ADP generated was then converted to ATP, and the ATP detected, using the AMP Detection Reagent. Briefly, cAMP titration with cAMP specific PDEs (amounts indicated on figures) was performed in a reaction buffer contains 40mM Tris-HCl, pH 7.5, 10mM MgCl₂, and 0.1mg/ml BSA. PDE reactions were carried out in 5µl for 60 min at 23°C. After incubation, 5ul of the AMP to ADP conversion reagent supplied with 500µM IBMX was added, mixed for 2 minutes on an orbital shaker and then incubated at room temperature for 60 minutes. To the samples, 10ul of AMP Detection Reagent was added, and the samples again incubated at room temperature for 60 minutes. Luminescence was then detected on a luminometer (Figure 4). The PDE reaction and the AMP to ADP conversion can be performed in one step by mixing the PDE, cAMP and the AMP-Glo Reagent I in 10µl reaction and incubating at room temperature for 60 minutes before adding the 10µl of AMP detection Reagent. The reaction was performed by in 384 well low volume plates (Coming Costar^{®} cat #3674).

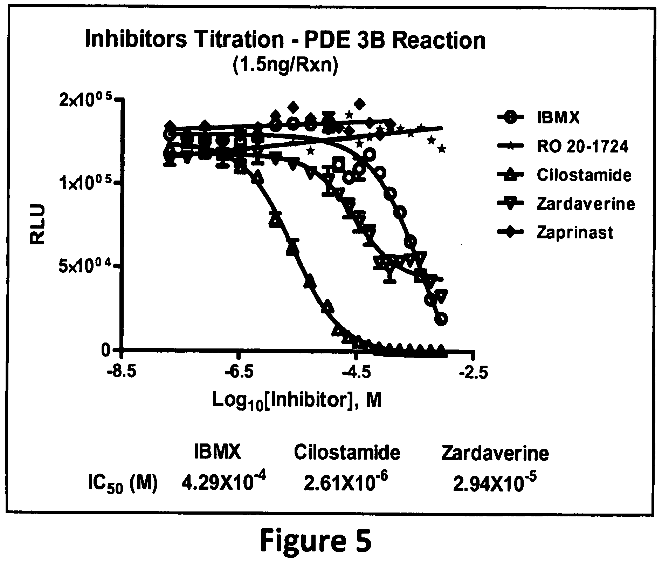

Reactions including different PDE 3B inhibitors were also performed as described above, and the IC50 concentration of inhibitors obtained with the method of the present invention was similar to that reported in the literature (1.5-2.0 µM) (Figure 5). The assay also showed very minimal false hits when screened against 1280 compounds of the LOPAC library, and it produced high Z' value confirming its robustness.

### Example 4: Bacterial and eukaryotic DNA Ligases

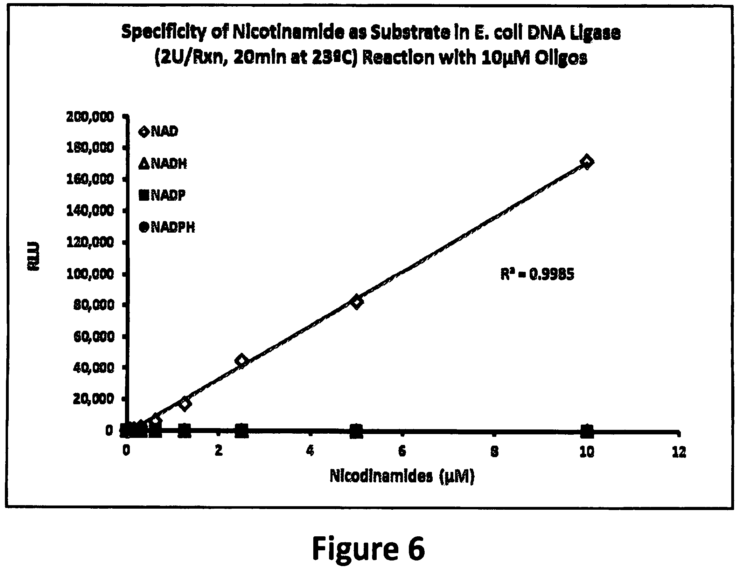

DNA ligases use NAD (bacterial) or ATP as substrate (viral and eukaryotic), but both enzymes generate AMP as one of the products. DNA ligation was demonstrated using E. Coli DNA ligase and synthetic oligos or lamda DNA Hind III fragments and in the presence of NAD (100 µM). *E. coli* DNA ligase reaction was performed in buffer containing 25mM Tris-HCl, pH 8.0, 5mM MgCl₂, 10mM (NH₄)₂SO₄, 1mM EDTA, and 0.1mg/ml BSA supplemented with different amounts of Nicotinamides (NAD, NADH, NADP and NADPH) 10µM Oligos using 384 well plate. The E. coli DNA ligase reaction was performed using 2U/reaction in 5µl volume and the reaction was incubated for 20 min at 23°C, followed by addition of 5µl of Reagent I for 60min at 23°C. After Reagent I reaction, 10µl of AMP Detection Solution was added and the luminescence was read after 60 min at 23°C. The results show that E.coli DNA ligase is dependent on NAD specifically as nicotinamide co-factor (Figure 6).

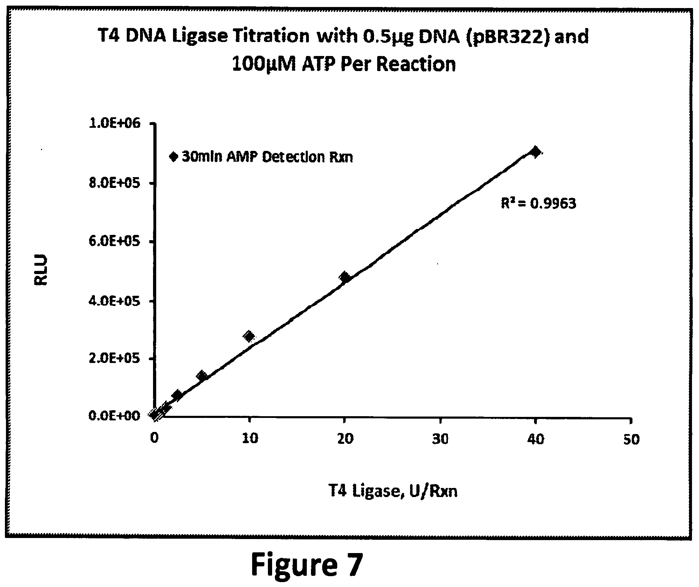

Similar assay conditions as described above were used to detect T4 DNA ligase (Promega) activity using an EcoRI-linearized vector (pBR322). T4 DNA ligase titration was carried out in a buffer contains 30mM Tris-HCl, pH 8.0, 10mM MgCl₂, 10mM DTT, 100µM ATP, in the presence of 0.5µg pBR322 vector that was pretreated with EcoR1 for 60 min. T4 DNA ligase reaction was performed in 10µl reaction for 30min at 23°C, then added 10µl of Reagent I for another 60min at 23°C. After Reagent I reaction, 20µl of AMP Detection Reagent was added and the luminescence was read after 60 min at 23°C (Figure 7).

### Example 5: AMP Determination in Cellular and Tissue Extracts

The method of the present invention is also used to determine the presence and/or amount of AMP in tissue extracts. After removal of any ATP present in the sample using the AMP Glo Reagent I, which also converted the AMP present to ADP, the ADP was converted to ATP, and the ATP detected using the AMP Detection Solution. The samples are then tested for conversion of AMP alone (containing PAP and AK) are compared to those which contained AK only and the difference was expressed as AMP.

### Example 6: Detection or quantitation of methyltransferases via AMP detection

Methyltransferases, e.g., DNA or protein methyltransferases, are used to carry out methylation of DNA or protein using S-adenosylmethionine (SAM) as substrate. Since S-adenosylmethionine (SAM) is the universal substrate, and S-adenosylhomocysteine (SAH) is the universal product for methyltransferases, the method of the present invention would involve the conversion of SAH to adenosine using SAH hydrolase, and the conversion of adenosine to AMP using adenosine kinase. The amount of AMP produced would be detected and/or determined using the method and compositions described herein.

### Quantitation of the different methyltransferase activities.

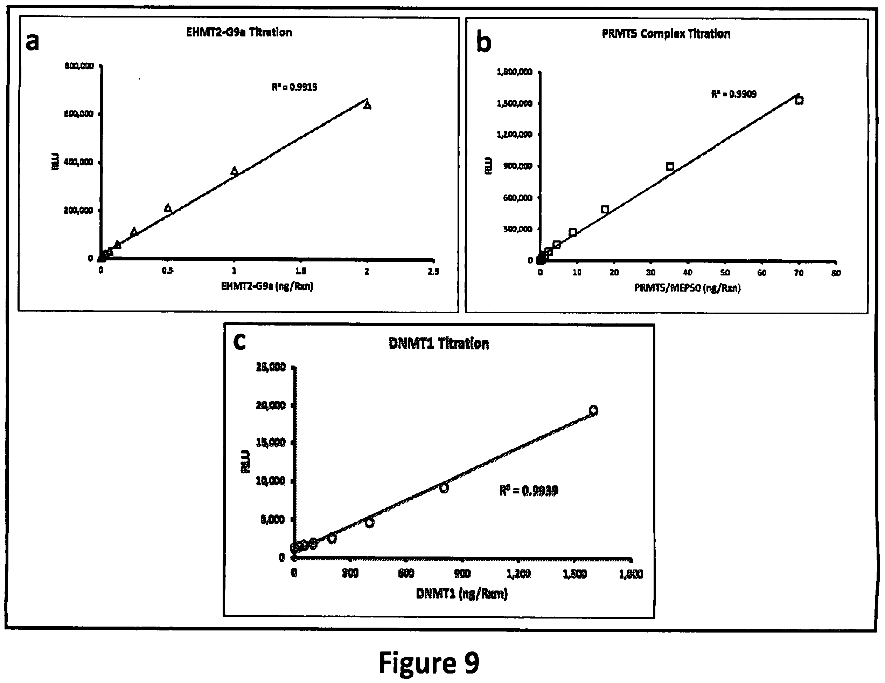

Methyltransferase reactions were performed in a reaction buffer (20mM Tri-HCl, pH 8.0, 50mM NaCl, 1mM EDTA, 3mM MgCl2, 1mM DTT, and 0.1mg/ml BSA) in the presence of different amounts of enzyme and the following substrate/ enzyme combinations: EHMT2-G9a with 50µM SAM and 50µM H3 peptide (1-25), PRMT5 complex with 50µM SAM and 50µM H4 (1-20) peptide, and DNMT1 with 10µM SAM and 0.1µM DNA oligos (Figure 9abc). All methyltransferase titration assays were carried out in 5µl reaction containing also the conversion enzymes SAH-Hydrolase and Adenosine Kinase with 25µM dGTP for 120min at 37°C, then 5µl of Reagent I was added for another 60min at 23°C. After Reagent I reaction, 20µl of AMP Detection Reagent was added and the luminescence was read after 60 min at 23°C (Figure 9).

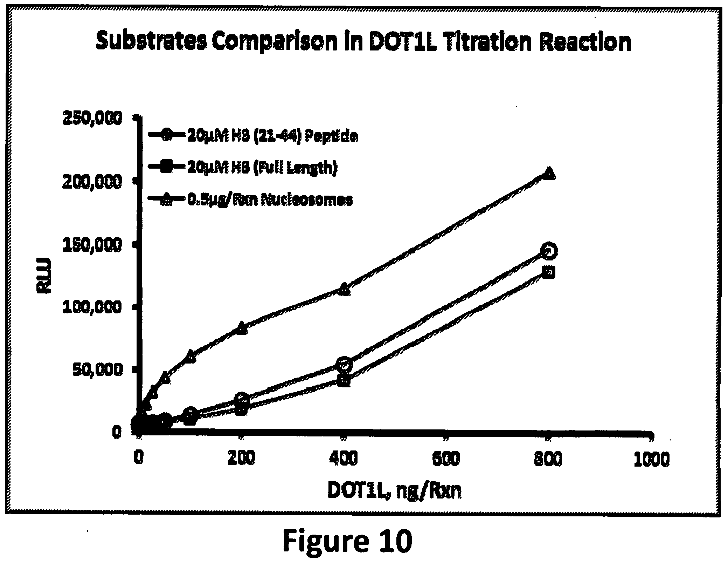

The DOT1L titration reaction with different substrates was performed in a reaction buffer (20mM Tri-HCl, pH 8.0, 50mM NaCl, 1mM EDTA, 3mM MgCl2, 1mM DTT, and 0.1mg/ml BSA) in the presence of 20µM SAM and either 20µM H3 (21-44) peptide, Histone H3 full length protein, or Nucleosomes. Similarly to the methyltransferase assay above, the DOT1L assay was carried out in 5µl reaction containing also the conversion enzymes SAH-Hydrolase and Adenosine Kinase with 25µM dGTP for 120min at 37°C, then 5µl of Reagent I was added for another 60min at 23°C. After Reagent I reaction, 20µl of AMP Detection Reagent was added and the luminescence was read after 60 min at 23°C (Figure 10).

### Example 7: Detection of deamidation of asparagine or rearrangement of aspartic acid residues in a protein

During long term storage and handling of proteins, a deamidation of asparagine residues and rearrangement of aspartic acid residues occur frequently at different levels resulting in their conversion to isoaspartate. In the biologies field it is very important to monitor the level of non-enzymatic deamidation of therapeutic proteins e.g. antibodies. To detect isoaspartate level on a protein, Protein Isoaspartyl Methyltransferase (PIMT) can be used to specifically detect the presence of isoaspartic acid residues in a target protein. PIMT transfers a methyl group from S-adenosyl-L-methionine (SAM) to isoaspartic acid, generating S-adenosyl homocysteine (SAH) that can be detected by the method and compositions of the present invention.

### Example 8: Aminoacyl tRNA-synthetase (ARS) Assay

The method and compositions of the present invention could be also used to monitor the activity of aminoacyl tRNA-synthetases (ARS) since ATP and other substrates are the components of the enzyme reaction, AMP is a product of this reaction, and the AMP generated from this reaction will be proportional to ARS activity.

The scope of the invention is defined by the appended claims.

## Claims

1. A method of determining the amount of adenosine monophosphate (AMP) in a solution comprising:
i) converting substantially all AMP in the solution to adenosine diphosphate (ADP) using a first enzyme that is capable of converting AMP to ADP, wherein the first enzyme is polyphosphate:AMP phosphotransferase (PAP);
ii) converting substantially all the ADP in the solution to adenosine triphosphate (ATP) using a second enzyme that is capable of converting ADP to ATP, wherein the second enzyme is adenylate kinase (AK);
iii) after step (ii) determining the amount of the ATP produced in step (ii) using a bioluminescent reaction utilizing a luciferase enzyme and a substrate for the luciferase enzyme.
iv) using the amount of ATP produced to determine the amount of AMP present in the solution before step (i);
wherein before step (i) there is a step of converting at least 95% of all adenosine triphosphate (ATP) in the solution to cyclic adenosine monophosphate (cAMP) and pyrophosphate with adenylate cyclase (AC).

2. The method of claim 1 wherein the solution further comprises polyphosphate in step (i).

3. The method of any one of claims 1 or 2 wherein step (ii) is simultaneous to step (i).

4. The method of any one of claims 1 or 2 wherein step (ii) is after step (i).

5. The method of any one of claims 1 to 4, further comprising before step (i) converting cAMP in the solution to AMP with a phosphodiesterase (PDE).

6. The method of any one of claims 1 to 4 further comprising simultaneous to step (i) converting cAMP in the solution to AMP using a phosphodiesterase (PDE).

7. The method of any one of claims 1 to 4 further comprising before step (i) converting ATP in the solution to AMP with a ubiquitinating enzyme and ubiquitin.

8. The method of any one of claims 1 to 4 further comprising before step (i) converting ATP in the solution to AMP with a DNA ligase.

9. The method of any one of claims 1 to 4 further comprising before step (i) converting nicotinamide adenine dinucleotide (NAD+) in the solution to AMP with a DNA ligase.

10. The method of claim 1 further comprising simultaneous to step (i) converting nicotinamide adenine dinucleotide (NAD+) in the solution to AMP with a DNA ligase.

11. The method of any one of claims 1 to 4 further comprising:
a) converting S-adenosyl methionine (SAM) in the solution to S-adenosylhomocysteine (SAH) using a methyltransferase;
b) converting the SAH to adenosine using an SAH hydrolase;
c) converting the adenosine to AMP using adenosine kinase.

12. The method of claim 11 wherein steps a), b) and c) are performed before step i).

13. The method of claim 11 wherein steps a), b) and c) are performed simultaneously to steps i) and ii).

14. The method of any one of claims 1 to 4 wherein before step (i) a step of converting ATP in the solution to AMP using an aminoacyl tRNA synthetase is performed.

15. The method of any one of claims 1 to 4 wherein the method further comprises removing substantially all pyrophosphate produced prior to steps (ii), (iii) and (iv).

16. The method of any one of claims 1 to 4 further comprising the step of inhibiting adenylate cyclase prior to, (ii) or (iii) with a composition comprising an inhibitor.

17. The method of any one of claims 1 to 4 wherein the adenylate cyclase is an active fragment of a full length bacterial adenylate cyclase or active recombinant fragment of a bacterial adenylate cyclase.

## Patentansprüche

1. Verfahren zum Bestimmen des Gehalts an Adenosinmonophosphat (AMP) in einer Lösung, Folgendes umfassend:
i) Umwandeln von im Wesentlichen dem gesamten AMP in der Lösung in Adenosindiphosphat (ADP) unter Einsatz eines ersten Enzyms, das in der Lage ist, AMP in ADP umzuwandeln, wobei das erste Enzym Polyphosphat:AMP-Phosphotransferase (PAP) ist;
ii) Umwandeln von im Wesentlichen dem gesamten ADP in der Lösung in Adenosintriphosphat (ATP) unter Einsatz eines zweiten Enzyms, das in der Lage ist, ADP in ATP umzuwandeln, wobei das zweite Enzym Adenylatkinase (AK) ist;
iii) nach Schritt (ii), Bestimmen des Gehalts an in Schritt (ii) erzeugtem ATP mit einer Biolumineszenzreaktion unter Einsatz eines Luziferaseenzyms und eines Substrats für das Luziferaseenzym.
iv) Verwenden des erzeugten Gehalts an ATP, um den Gehalt an vor Schritt (i) in der Lösung enthaltenem AMP zu bestimmen;
wobei vor Schritt (i) ein Schritt des Umwandeins von wenigstens 95 % des gesamten Adenosintriphosphats (ATP) in der Lösung in cyclisches Adenosinmonophosphat (cAMP) und Pyrophosphat mit Adenylatcyclase (AC) stattfindet.

2. Verfahren nach Anspruch 1, wobei die Lösung ferner Polyphosphat in Schritt (i) umfasst.

3. Verfahren nach einem der Ansprüche 1 oder 2, wobei Schritt (ii) gleichzeitig mit Schritt (i) stattfindet.

4. Verfahren nach einem der Ansprüche 1 oder 2, wobei Schritt (ii) nach Schritt (i) stattfindet.

5. Verfahren nach einem der Ansprüche 1 bis 4, ferner vor Schritt (i) das Umwandeln von cAMP in der Lösung mit einer Phosphodiesterase (PDE) in AMP umfassend.

6. Verfahren nach einem der Ansprüche 1 bis 4, ferner gleichzeitig mit Schritt (i) das Umwandeln von cAMP in der Lösung unter Einsatz einer Phosphodiesterase (PDE) in AMP umfassend.

7. Verfahren nach einem der Ansprüche 1 bis 4, ferner vor Schritt (i) das Umwandeln von ATP in der Lösung mit einem ubiquitinierenden Enzym und Ubiquitin in AMP umfassend.

8. Verfahren nach einem der Ansprüche 1 bis 4, ferner vor Schritt (i) das Umwandeln von ATP in der Lösung mit einer DNA-Ligase in AMP umfassend.

9. Verfahren nach einem der Ansprüche 1 bis 4, ferner vor Schritt (i) das Umwandeln von Nicotinamidadenindinukleotid (NAD+) in der Lösung mit einer DNA-Ligase in AMP umfassend.

10. Verfahren nach Anspruch 1, ferner gleichzeitig mit Schritt (i) das Umwandeln von Nicotinamidadenindinukleotid (NAD+) in der Lösung mit einer DNA-Ligase in AMP umfassend.

11. Verfahren nach einem der Ansprüche 1 bis 4, ferner Folgendes umfassend:
a) Umwandeln von S-Adenosylmethionin (SAM) in der Lösung unter Einsatz einer Methyltransferase in S-Adenosylhomocystein (SAH);
b) Umwandeln des SAH unter Einsatz einer SAH-Hydrolase in Adenosin;
c) Umwandeln des Adenosins unter Einsatz von Adenosinkinase in AMP.

12. Verfahren nach Anspruch 11, wobei Schritte a), b) und c) vor Schritt i) durchgeführt werden.

13. Verfahren nach Anspruch 11, wobei Schritte a), b) und c) gleichzeitig mit Schritten i) und ii) durchgeführt werden.

14. Verfahren nach einem der Ansprüche 1 bis 4, wobei vor Schritt (i) ein Schritt des Umwandelns von ATP in der Lösung unter Einsatz einer Aminoacyl-tRNA-Synthetase in AMP durchgeführt wird.

15. Verfahren nach einem der Ansprüche 1 bis 4, wobei das Verfahren ferner das Entfernen von im Wesentlichen dem gesamten erzeugten Pyrophosphat vor den Schritten (ii), (iii) und (iv) umfasst.

16. Verfahren nach einem der Ansprüche 1 bis 4, ferner umfassend den Schritt des Hemmens von Adenylatcyclase vor (ii) oder (iii) mit einer Zusammensetzung, die einen Hemmer umfasst.

17. Verfahren nach einem der Ansprüche 1 bis 4, wobei die Adenylatcyclase ein aktives Fragment einer bakteriellen Volllängen-Adenylatcyclase oder ein aktives rekombinantes Fragment einer bakteriellen Adenylatcyclase ist.

## Revendications

1. Méthode permettant de déterminer la quantité d'adénosine monophosphate (AMP) dans une solution, comprenant :
i) la conversion d'essentiellement tout l'AMP dans la solution en adénosine diphosphate (ADP) en utilisant une première enzyme qui est capable de convertir l'AMP en ADP, où la première enzyme est la polyphosphate-AMP phosphotransférase (PAP) ;
ii) la conversion d'essentiellement tout l'ADP dans la solution en adénosine triphosphate (ATP) en utilisant une deuxième enzyme qui est capable de convertir l'ADP en ATP, où la deuxième enzyme est une adénylate kinase (AK) ;
iii) après l'étape (ii), la détermination de la quantité d'ATP produite à l'étape (ii) au moyen d'une réaction de bioluminescence utilisant une enzyme de type luciférase et un substrat de l'enzyme de type luciférase ;
iv) l'utilisation de la quantité d'ATP produite pour déterminer la quantité d'AMP présente dans la solution avant l'étape (i) ;
où une étape de conversion d'au moins 95 % de toute l'adénosine triphosphate (ATP) dans la solution en adénosine monophosphate cyclique (AMPc) et en pyrophosphate avec une adénylate cyclase (AC) est effectuée avant l'étape (i).

2. Méthode selon la revendication 1 où, à l'étape (i), la solution comprend en outre un polyphosphate.

3. Méthode selon l'une quelconque des revendications 1 ou 2, où l'étape (ii) est effectuée en même temps que l'étape (i).

4. Méthode selon l'une quelconque des revendications 1 ou 2, où l'étape (ii) est effectuée après l'étape (i).

5. Méthode selon l'une quelconque des revendications 1 à 4, qui comprend en outre la conversion de l'AMPc dans la solution en AMP avec une phosphodiestérase (PDE) avant l'étape (i).

6. Méthode selon l'une quelconque des revendications 1 à 4, qui comprend en outre la conversion de l'AMPc dans la solution en AMP avec une phosphodiestérase (PDE) en même temps que l'étape (i).

7. Méthode selon l'une quelconque des revendications 1 à 4 qui comprend en outre la conversion de l'ATP dans la solution en AMP avec une enzyme d'ubiquitylation et l'ubiquitine avant l'étape (i).

8. Méthode selon l'une quelconque des revendications 1 à 4, qui comprend en outre la conversion de l'ATP dans la solution en AMP avec une ADN ligase avant l'étape (i).

9. Méthode selon l'une quelconque des revendications 1 à 4, qui comprend en outre la conversion du nicotinamide adénine dinucléotide (NAD⁺) dans la solution en AMP avec une ADN ligase avant l'étape (i).

10. Méthode selon l'une quelconque des revendications 1 à 4, qui comprend en outre la conversion du nicotinamide adénine dinucléotide (NAD⁺) dans la solution en AMP avec une ADN ligase en même temps que l'étape (i).

11. Méthode selon l'une quelconque des revendications 1 à 4, qui comprend en outre :
a) la conversion de la S-adénosyl méthionine (SAM) dans la solution en S-adénosyl homocystéine (SAH) en utilisant une méthyltransférase ;
b) la conversion de la SAH en adénosine en utilisant une SAH hydrolase ;
c) la conversion de l'adénosine en AMP en utilisant une adénosine kinase.

12. Méthode selon la revendication 11, où les étapes a), b) et c) sont effectuées avant l'étape (i).

13. Méthode selon la revendication 11, où les étapes a), b) et c) sont effectuées en même temps que les étapes (i) et (ii).

14. Méthode selon l'une quelconque des revendications 1 à 4, où une étape de conversion de l'ATP dans la solution en AMP en utilisant une aminoacyl-tARN synthétase est effectuée avant l'étape (i).

15. Méthode selon l'une quelconque des revendications 1 à 4, où la méthode comprend en outre l'élimination d'essentiellement tout le pyrophosphate produit avant les étapes (ii), (iii) et (iv).

16. Méthode selon l'une quelconque des revendications 1 à 4, qui comprend en outre l'étape d'inhibition de l'adénylate cyclase avec une composition comprenant un inhibiteur avant l'étape (ii) ou (iii).

17. Méthode selon l'une quelconque des revendications 1 à 4, où l'adénylate cyclase est un fragment actif d'une adénylate cyclase bactérienne de pleine longueur ou un fragment recombinant actif d'une adénylate cyclase bactérienne.


### SEQUENCE LISTING

<110> PROMEGA CORPORATION
<120> METHODS FOR DETECTING ADENOSINE MONOPHOSPHATE IN BIOLOGICAL SAMPLES
<130> 016026-9548-US01
<140> New Patent Application
   <141> 2012-10-29
<150> US 61/552,837
   <151> 2011-10-28
<160> 2
<170> PatentIn version 3.5
<210> 1
   <211> 30
   <212> DNA
   <213> Artificial Sequence
<220>
   <223> Synthetic oligonucleotide
<400> 1
   aagtttaaac ttaacgcccg ctttggttta 30
<210> 2
   <211> 30
   <212> DNA
   <213> Artificial Sequence
<220>
   <223> Synthetic oligonucleotide
<400> 2
   aagcgatcgc acggaactcg ctatcacaaa 30